# EUROPÄISCHE PATENTSCHRIFT

(11) **EP 2 210 562 B1**
(45) Veröffentlichungstag und Bekanntmachung des Hinweises auf die Patenterteilung: **20.03.2013**
(21) Anmeldenummer: 10000236.9
(22) Anmeldetag: 12.01.2010
(51) Int. Cl.: A61B 8/00

(54) **Geführte Sonografie**
Guided sonography
Sonographie pilotée

(30) Priorität: 22.01.2009 DE 102009005707
(43) Veröffentlichungstag der Anmeldung: 28.07.2010
(73) Patentinhaber: Pohlig GmbH, 83278 Traunstein (DE)
(72) Erfinder: Pohlig, Kurt, 83278 Traunstein (DE)
(74) Vertreter: Paustian, Othmar

(56) Entgegenhaltungen:
- EP-A1- 1 498 081
- WO-A1-98/38908
- US-A1- 2002 088 926
- US-A1- 2009 234 370

## Beschreibung

Die Erfindung betrifft ein System zur geführten Sonografie sowie einen austauschbaren Träger für ein entsprechendes System.

Sonografie ist an sich allgemein bekannt; es handelt sich um die Anwendung von Ultraschall zur Abbildung.

In Medizin und Veterinärmedizin wird die Sonografie regelmäßig zur Untersuchung von Knochen, Gelenken und Organen eingesetzt. Es ist an sich auch bekannt, andere Materialien zur Darstellung ihrer inneren Struktur zu sonografieren.

In der Kinderorthopädie wird die Sonografie flächendeckend zur Untersuchung der Hüfte eingesetzt. Es werden unter anderem Überdachungsdefizite und diverse anatomische Winkel ausgemessen. Für Kinderknochen bietet sich die Sonografie besonders an, da diese noch nicht vollständig durchgekalkt sind und ggf. nicht gut im Röntgenbild zu erkennen sind.

Zur Abbildung wird der Ultraschallkopf an die Oberfläche eines zu sonografierenden Objekts angelegt, etwa an ein Körperteil zur Abbildung der im Inneren des Körperteils liegenden Knochen. Dabei wird der Schallkopf von einer Bedienperson manuell an die Oberfläche des Objekts angelegt und ggf. auch an ihr entlang geführt.

Die resultierende Abbildung hängt stark von der Lage, also Ausrichtung und Position, des angelegten Schallkopfs ab. Fehlerfreie Abbildungsergebnisse werden nur erzielt, wenn der Schallkopf gegenüber seiner optimalen Ausrichtung nicht verkippt oder verdreht ist; außerdem kann der Schallkopf bei der Führung entlang des Objekts auch verkanten. Durch Verkippen, Verdrehen oder Verkanten des Schallkopfes ergeben sich topografische Übertragungsfehler der Schallkopf-Position auf die Körperoberfläche. Das birgt die Gefahr erheblicher Messdifferenzen, insbesondere bei ungeübten Anwendern.

Gerade im medizinischen Bereich ist eine optimale Abbildungsqualität von hoher Relevanz.

Beispielsweise ist bei der sonografischen Bestimmung der distalen Wachstumsfuge am Oberschenkel die Position der Wachstumsfuge von Interesse. Hier wird beispielsweise bei der Verwendung eines Schallkopfes mit paralleler Abstrahlcharakteristik (sog. Linearschallkopf) wie folgt vorgegangen: Befindet sich die Wachstumsfuge in der Abbildung in der Mitte, so wird auch die Mitte des Schallkopfes auf die Haut übertragen, was die Position der Wachstumsfuge nach außen sichtbar machen soll. Dieses wird auch als topografische Übertragung auf die Körperoberfläche bezeichnet. Ist der Schallkopf jedoch nicht korrekt ausgerichtet, wird diese Position falsch markiert.

Besonders schwerwiegend kann etwa eine fehlerhafte Bestimmung des Pfannendachwinkels der Hüfte sein; hier kann eine fehlerhafte Bestimmung des Winkels sogar zu einer unnötigen Operation führen.

Die Anfertigung und/oder Interpretation sonografischer Abbildungen erfordert viel Erfahrung. Die zugehörigen unterschiedlichen Fähigkeiten sind schwer zu erlernen. Die Qualität der Abbildungen hängt von vielen Umständen ab, die zumindest teilweise gleichzeitig berücksichtigt werden müssen, beispielsweise: Position und Lage des Objekts, etwa eines Patienten, Geräteeinstellungen und vor allem die Lage und Handhabung des Schallkopfes. Dies setzt eine gute Hand-Auge-Koordination voraus. Der subjektive Eindruck muss dann mit dem erwarteten Ergebnis verglichen und Abweichungen hinsichtlich ihrer Bedeutung beurteilt werden; dies setzt einiges an Erfahrung voraus. Entsprechend sind die Ergebnisse auch nur in begrenztem Umfang objektiv.

Korrekte Abbildungen können verlässlich nur von erfahrenen und fähigen Bedienpersonen erstellt werden.

Der Erfindung liegt die Aufgabe zu Grunde, ein vorteilhaftes System zur Sonografie anzugeben, welches die Anforderungen an eine Bedienperson verringert.

Die Aufgabe wird gelöst durch ein System zur geführten Sonografie mit einem Ultraschallkopf, der an die Oberfläche eines zu sonografierenden Objekts anlegbar und dort in seiner Lage veränderbar ist, einem Projektor zur Projektion einer Markierung auf das zu sonografierende Objekt, einer Kopplungseinrichtung, die die Projektionen an die Lage des Ultraschallkopfes koppelt, wobei die Markierung eine vorgegebene Position und/oder Ausbildung auf dem zu sonografierenden Objekt aufweist, wenn der Ultraschallkopf an diesem korrekt anliegt.

Bevorzugte Ausgestaltungen der Erfindung sind in abhängigen Ansprüchen angegeben und werden im Folgenden ebenfalls näher erläutert.

Die Erfindung beruht auf der Idee, der Bedienperson eine Rückmeldung betreffend der korrekten Lage des Schallkopfes anzubieten; in diesem Sinne handelt es sich um eine "geführte" Sonografie.

Die Führung drückt sich durch Position und/oder Ausbildung der Markierung auf dem zu sonografierenden Objekts aus. Ist der Schallkopf korrekt angelegt, so ist dies an der Markierung zu erkennen. Ist der Schallkopf nicht korrekt angelegt, so ist dies ebenfalls an der Markierung zu erkennen; sie nimmt in diesem Fall eben nicht die vorgegebene Position und/oder Ausbildung an.

Der Projektor dient zur Projektion der Markierung auf das zu sonografierende Objekt. Bei dem Projektor kann es sich etwa um eine gerichtete Lichtquelle und insbesondere um einen Laser handeln.

Die Projektion des Projektors ist von der Lage des Ultraschallkopfes abhängig. Dazu umfasst die Erfindung eine Kopplungseinrichtung, die gewährleistet, dass sich die Lage des Schallkopfes auf die Projektion auswirkt. Im einfachsten Fall bewirkt eine Lageänderung des Schallkopfes eine Lageänderung des Projektors und damit etwa eine Richtungsänderung der Projektion, was zu einer Änderung der Markierung auf dem interessierenden Objekt führt; dazu kann die Kopplungseinrichtung eine elektrische oder elektronische Einrichtung aufweisen oder eine mechanische, den Schallkopf und den Projektor verbindende Einrichtung sein, etwa ein beide Teile tragender Träger (vgl. entsprechende Ausgestaltungen unten).

Erfindungsgemäße Projektionen bzw. Markierungen können unterschiedlich gestaltet sein. Es sind geometrische Projektionen, aber auch Projektionen anderer Qualität denkbar. So kann es sinnvoll sein, die Lage des Schallkopfes ausschließlich oder auch zusätzlich farblich oder auch über projizierte Symbole auszudrücken. Es ist jede Projektion sinnvoll, die es erlaubt, eine Aussage über einen Aspekt der Lage des Ultraschallkopfes zu machen.

Zur Veranschaulichung sei auf eine Projektion verwiesen, bei der es sich um ein mit einem Laser projiziertes Kreuz mit zwei sich gegenseitig mittig kreuzenden, gleich langen und orthogonal zueinander liegenden Balken handelt. Das erfindungsgemäße System kann hier etwa so eingerichtet werden, dass bei einem korrekt ausgerichteten Schallkopf die Markierung, also das Kreuz, auf der Oberfläche des Objekts symmetrisch und bei einem verkippten Schallkopf verzerrt ist. Auf diese Weise kann eine Bedienperson leicht sehen, ob sie den Schallkopf korrekt aufgesetzt hat. Außerdem kann die kreuzförmige Markierung noch vorteilhaft in Bezug zu Referenzpunkten/ oder -linien an der Oberfläche des Objekts gesetzt werden, was zusätzlich auch eine einfache Kontrolle der Position des Schallkopfes erlaubt.

Die Referenzen können bspw. aufgemalt oder ebenfalls projiziert oder auf der Oberfläche des Objekts vorhandene natürliche markante Orientierungshilfen sein.

Der Ultraschallkopf kann beispielsweise, wie bereits erwähnt, ein linearer Schallkopf, oder aber etwa auch ein Sektorenschallkopf sein (ein Sektorenschallkopf erzeugt ein divergierendes Schallfeld). Mit einem Sektorenschallkopf werden typischerweise innere Organe abgebildet. Sektorenschallköpfe sind eher darauf ausgelegt, einen größeren Winkelbereich abzubilden, als eine genaue Lokalisation zu ermöglichen. Die Erfindung erleichtert die Lokalisation bzw. die Markierung relevanter Positionen auf der Haut auch mit einem Sektorenschallkopf, da die Lage des Schallkopfs über die projizierte Markierung kontrolliert werden kann.

Eine Bedienperson kann sich bei einem System nach der Erfindung durch die Markierung führen lassen. Je nach Ausgestaltung der Erfindung kann die Markierung einen oder mehrere Aspekte der Lage des Schallkopfes ausdrücken. Insgesamt ermöglicht die Erfindung eine Rückmeldung bezüglich aller Aspekte der Lage, also die Ausrichtung und Position im Raum inklusive Position an der Oberfläche des zu sonografierenden Objekts. Damit wird es auch unerfahrenen Bedienpersonen erleichtert, qualitativ hochwertige Aufnahmen anzufertigen.

In der technischen Orthopädie lassen sich mit der Erfindung beispielsweise besonders vorteilhaft relevante Daten für die Konstruktion von Orthesen erzeugen. Hier sind z.B. die mechanischen Drehachsen oder die anatomische Höhe von Gelenken, etwa des Chopart-Gelenks im Fuß, von Interesse.

Beispielsweise kann mit dem System nach der Erfindung besonders vorteilhaft die distale Wachstumsfuge (femorale Epiphyse) am Oberschenkelknochen (Femur) über die mediale und laterale Seite des Beins bestimmt werden. In Höhe der Wachstumsfuge befindet sich der anatomische (Kompromiss-) Drehpunkt des Kniegelenks. Aus diesen Daten lässt sich schließen, in welcher anatomischen Höhe der mechanische Gelenkschienendrehpunkt an einer das Knie übergreifenden Orthese positioniert werden sollte.

Die Erfindung ermöglicht eine exaktere Übertragung von anatomischen Gelenklinien, Wachstumsfugen, Gelenkdrehpunkte u.s.w. auf die Körperoberfläche bzw. auf deren Topografie. Dies eröffnet beispielsweise dem Orthopädietechniker bisher nicht bekannte Möglichkeiten zur anatomisch akkuraten Anpassung von orthopädischen Hilfsmitteln.

Beispielsweise kann das Gerät aber auch vorteilhaft zur Festlegung von durchzuführenden Schnitten in die Haut bei Operationen oder aber auch zur Festlegung der örtlichen Ausgestaltung von Bestrahlungen eingesetzt werden.

Besonders vorteilhaft ist auch die Möglichkeit, bei der menschlichen Wirbelsäule die Rotation der Wirbel ohne Röntgen, also nicht-invasiv, zu vermessen. Die Erfindung erlaubt es, die Abweichung der Wirbel von einer als Referenz fungierenden Senkrechten besonders genau zu vermessen.

Bei einer bevorzugten Ausführungsform der Erfindung ändert sich die Markierung in Abhängigkeit von der Lage des Ultraschallkopfes aufgrund einer Änderung der Projektionsrichtung. In anderen Worten: Eine Lageänderung des Ultraschallkopfes führt zu einer Änderung der Projektionsrichtung. Entsprechende Ausführungsformen sind konzeptionell und konstruktiv besonders einfach, da die Projektion an sich, also etwa das oben bereits erwähnte Laserkreuz, unveränderlich sein kann und lediglich die Projektionsrichtung geändert wird. Dies kann durch eine Änderung der Ausrichtung des Projektors erzielt werden.

Bei dem Projektor handelt es sich vorzugsweise um einen Laser.

Vorzugsweise wird, wie oben bereits erwähnt, ein Kreuz projiziert. Ein Kreuz kann besonders leicht an einer Referenzlinie entlanggeführt werden. Dabei wird beispielsweise der erste Balken parallel zu der Referenzlinie geführt, und der dazu senkrechte zweite Balken erlaubt eine Aussage über die Position des Schallkopfs entlang der Referenzlinie. Werden interessante innere Strukturen mit dem Schallkopf gefunden, unterstützt der zweite Balken eine korrekte Markierung der Position auf der Oberfläche des Objekts. Ist der erste Balken zwar parallel zu der Referenzlinie ausgerichtet, von dieser jedoch beabstandet, kann dies für ein Verkippen des Schallkopfes quer zu der Referenzlinie sprechen (vgl. Beispiel).

Vorstellbar ist beispielsweise auch eine kreisrunde Projektion; ist der Ultraschallkopf verkippt, so ergibt sich etwa eine ovale Markierung. Auch mit einer einfachen kurzen Linie als Markierung kann schon sinnvoll gearbeitet werden, man kann etwa auf die Ausrichtung relativ zu einer Referenzlinie bzw. einen Abstand von einer solchen abstellen.

Insgesamt kann es wünschenswert sein, die relative Lage von dem Projektor zu dem Schallkopf veränderlich und lösbar feststellbar auszulegen. So kann das System durch unterschiedliche Einstellungen an unterschiedliche Umstände angepasst werden. Dies betrifft nicht nur Systeme mit einem Träger für den Ultraschallkopf und den Projektor, sondern ist auch für Systeme ohne Träger vorteilhaft.

Bei einer bevorzugten Ausführungsform der Erfindung umfasst die Kopplungseinrichtung eine Messeinrichtung zur Messung der Lage des Schallkopfs und eine Steuereinrichtung zur Steuerung des Projektors basierend auf der Messung der Lage des Schallkopfs.

Bei der Messeinrichtung kann es sich etwa um Lagesensoren handeln. Die resultierenden Daten können über eine Kommunikationseinrichtung, etwa über Funk, WLAN oder LAN an eine Steuereinrichtung, beispielsweise umfassend einen konventionellen, entsprechend programmierten Rechner, übermittelt werden. Die Steuereinrichtung wirkt schließlich, gegebenenfalls mittelbar über Aktuatoren, etwa Elektromotoren, auf den Projektor ein.

Vorzugsweise wird die Lage des Schallkopfes zur visuellen Wahrnehmung zusätzlich dargestellt, beispielsweise auf einem Monitor. Die zur Visualisierung erforderlichen Daten über die Lage des Schallkopfes können etwa mit den eben bereits erwähnten Lagesensoren erzeugt werden.

Besonders bevorzugt ist die Durchführung einer Sonografie mit einem System nach der Erfindung vermittels einer virtuellen Umgebung. Hier kann die Bedienperson, beispielsweise über einen Datenhandschuh, den virtuellen Gegenpart des Schallkopfes in der virtuellen Welt virtuell bewegen und sich dabei durch die virtuelle Markierung auf einem virtuellen Objekt führen lassen. Auf diese Weise ließen sich die Systeme nach der Erfindung auch vorteilhaft fernsteuern. Es müsste lediglich noch für eine geeignete Aufhängung und Ansteuerung des realen Schallkopfes gesorgt werden.

Besonders bevorzugt ist es, den Projektor und den Ultraschallkopf an einem gemeinsamen Träger zu befestigen, so dass eine Lageänderung des Ultraschallkopfes über den Träger eine Lageänderung der Projektion bewirkt. Eine entsprechende mechanische Kopplung ist konstruktiv besonders einfach und auch intuitiv zu bedienen. Außerdem können entsprechende Ausgestaltungen der Erfindung vergleichsweise klein gebaut werden, so dass die Geräte gut transportiert werden können bzw. mobil einsetzbar sind.

Bei dem Träger handelt es sich vorzugsweise um einen U-förmigen Bügel. Der Projektor kann an einem Ende des Bügels und der Ultraschallkopf an dem anderen Ende des Bügels befestigt sein. Aufgrund der U-Form kann der Bügel um einen Teil des zu sonografierenden Objektes herumgreifen. Beispielsweise ist es möglich, das Knie von der Seite her zu sonografieren und dabei eine auf die Kniescheibe projizierte Markierung mit einer Referenz abzugleichen. Je nach Größe und Form des zu sonografierenden Objekts/Körperteils kann der Bügel entsprechend angepasst, mal größer oder mal kleiner oder mit einem anderen Krümmungsradius, sein. Insbesondere lassen sich so gut Ausführungen realisieren, die handlich sind und gut manuell bedient werden können bzw. auch für den mobilen Einsatz taugen.

Unter dem Begriff "U-förmiger Bügel" werden hier einstückige und mehrteilige Konstruktionen verstanden, die z. B. verrundet, etwa als verrundet gebogener Bügel, oder polygonzugartig ausgebildet sein können. So sind auch Bügel denkbar, die etwa aus mehreren, beispielsweise drei, geraden Schienen bestehen. Diese können etwa im rechten Winkel miteinander in Verbindung stehen und dabei über Langlöcher auch gegeneinander verschoben werden. Auch mehr als drei gerade Schienen sind grundsätzlich denkbar.

Vorzugsweise ist das System so ausgelegt, dass der Träger austauschbar ist. Dies erleichtert die Verwendung von Trägem mit unterschiedlicher Geometrie oder Eigenschaften. Beispielsweise kann in einem Fall ein starrer Träger besonders vorteilhaft sein, während in einem anderen Fall ein biegsamer Schwanenhals gewünscht ist.

Weiter ist es vorteilhaft, den Projektor verschiebbar und an verschiedenen Positionen lösbar fixierbar an dem Träger zu befestigen. So kann das System auch ohne Austausch des Trägers an das zu sonografierende Objekt angepasst werden.

Weiter ist es bevorzugt, eine Referenzmarkierung mit einem zweiten Projektor auf das zu sonografierende Objekt zu projizieren. Im einfachsten Fall kann es sich hier um eine Referenzlinie handeln. Eine entsprechende Projektion ist zuverlässiger als eine gedachte Referenz und muss, anders als eine aufgemalte Referenz, auch nicht wieder abgewaschen werden.

Die Erfindung betrifft auch einen Träger, insbesondere einen Arm oder Bügel, für ein System zur geführten Sonografie, um dieses an bestimmte Anwendungsumstände anzupassen. Entsprechende Träger sind so ausgelegt dass sie einen Projektor und einen Ultraschallkopf so aufnehmen können, dass Systeme nach der Erfindung damit gebildet werden können. Die Träger können die unterschiedlichsten Geometrien und andere Eigenschaften haben. Die Erfindung betrifft grundsätzlich auch die Verwendung eines erfindungsgemäßen Trägers als Austauschteil in einem erfindungsgemäßen System.

Die vorangehende und die folgende Beschreibung bezieht sich sowohl auf das System als auch auf den Träger, ohne dass dies immer explizit ausformuliert ist; die dabei offenbarten Merkmale können auch einzeln und in anderen als den gezeigten Kombinationen erfindungsgemäß sein.

Im Folgenden soll die Erfindung auch anhand von Ausführungsbeispielen näher erläutert werden, ohne dabei die Erfindung durch diese Beispiele einschränken zu wollen:
Figur 1 zeigt eine Prinzipskizze einer ersten Ausführungsform des erfindungsgemäßen Systems.
Figur 2 zeigt eine Prinzipskizze, anhand der zwei Bewegungsfreiheitsgrade des Systems aus Figur 1 veranschaulicht werden.
Figur 3 zeigt eine Markierung nach der Erfindung mit einer Referenzlinie.
Figur 4 zeigt eine Prinzipskizze einer zweiten Ausführungsform des erfindungsgemäßen Systems.
Figur 5 zeigt die Verwendung einer Ausführungsform entsprechend den Figuren 1 und 2 an einem Knie.

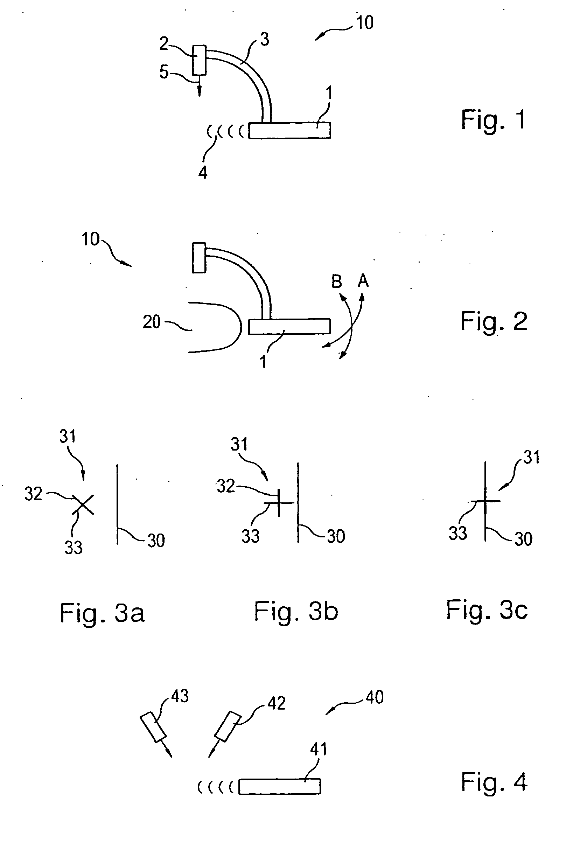

Figur 1 zeigt ein erstes erfindungsgemäßes System 10. Dieses umfasst einen linearen Ultraschallkopf 1, einen Laser 2 und einen Bügel 3. Der Ultraschallkopf 1 und der Laser 2 sind jeweils an einem Ende des Bügels 3 befestigt. Links von dem Ultraschallkopf 1 ist ein Schallfeld 4 angedeutet. Der Pfeil 5 unterhalb des Lasers 2 deutet die Projektionsrichtung des Lasers 2 an.

Der Ultraschallkopf 1 ist dazu ausgelegt, an die Oberfläche eines zu sonografierenden Objektes (hier nicht gezeigt) anlegbar zu sein. Dabei ist er in seiner Lage veränderbar. Beispielsweise kann das System 10 mit der Hand gegriffen und geführt werden.

Der Laser 2 projiziert 5 eine Markierung auf das zu sonografierende Objekt (nicht gezeigt).

Der Bügel 3 koppelt den Ultraschallkopf 1 und den Laser 2 derart aneinander, dass eine Lageänderung des Ultraschallkopfes 1 eine Lageänderung des Lasers 2 verursacht. Dabei wird auch die Richtung der Projektion 5 geändert, was sich auf die Markierung auf dem zu sonografierenden Objekt auswirkt.

Der Laser 2 und/oder der Ultraschallkopf 1 können auch entlang des Bügels 3 verschiebbar und lösbar fixierbar sein. So kann die Geometrie des Systems 10 leicht an die Proportionen des zu sonografierenden Objekts angepasst werden.

Der Bügel 3 kann gegen einen anderen Bügel mit einer anderen Geometrie ausgetauscht werden. Dies verbessert weiter die Anpassbarkeit des Systems 10.

Figur 2 zeigt anhand einer Prinzipskizze die Anlage des Systems 10 an einen Oberschenkel 20. Der Ultraschallkopf 1 muss dabei nicht unmittelbar auf der Haut des Oberschenkels 20 aufliegen, gegebenenfalls kann etwa ein Kontaktgel in einem Spalt zwischen dem Ultraschallkopf 1 und dem Oberschenkel 20 gegeben sein.

Die Pfeile A und B deuten die Verkippungsfreiheitsgrade des Systems 10 an. Zusätzlich kann das System 10 natürlich auch an der Oberfläche des Oberschenkels 20 entlanggeführt werden, also translatorisch und/oder rotatorisch bewegt werden. Die Figuren 3a, 3b und 3c zeigen jeweils eine Referenzlinie 30 und eine projizierte Markierung 31, hier ein Kreuz. Die Referenzinie 30 ist auf das zu sonografierende Objekt aufgemalt. Sie ist dabei so gewählt und das System nach der Erfindung so ausgelegt, dass das System korrekt ausgerichtet ist, wenn der erste Balken 32 des Kreuzes 31 auf die Referenzlinie 30 fällt und dabei der zweite Balken 33 senkrecht dazu steht.

In Teilfigur a) liegen zwei Abweichungen vor, einmal ist das Kreuz 31 gegenüber der Referenzlinie 30 verdreht und ausserdem ist der Schnittpunkt des Kreuzes 31 von der Referenzlinie 30 beabstandet. Der Ultraschallkopf muss nun so bewegt werden, dass beide Abweichungen nivelliert werden. In Teilfigur b) ist der erste Balken 32 parallel zu der Referenzlinie ausgerichtet, der Schnittpunkt des Kreuzes 31 ist allerdings immer noch von dieser Referenzlinie 30 beabstandet. In Teilfigur c) fällt schließlich das Kreuz 31 auf die Referenzlinie 30, dabei steht der zweite Balken 33 senkrecht auf der Referenzlinie 30.

Das erfindungsgemäße System kann so ausgelegt sein, dass der zweite Balken 33 auch die Mitte des Ultraschallbildes angibt. Durchläuft eine interessante Struktur die Abbildung, so kann sie auf der Haut entsprechend leicht markiert werden.

Figur 4 zeigt ein weiteres erfindungsgemäßes System 40. Auch dieses weist einen Ultraschallkopf 41 und einen Laser 42 auf. Zusätzlich umfasst dieses System 40 einen zweiten Laser 43. Der erste Laser 42 dient zur Projektion der Markierung auf das zu sonografierende Objekt, welche über eine Kopplungseinrichtung (nicht gezeigt) an den Ultraschallkopf 41 gekoppelt ist. Der zweite Laser 43 dient zur Projektion einer Referenzlinie auf das zu sonografierende Objekt. Die Projektion des zweiten Lasers 43 ist unabhängig von der Lage des Ultraschallkopfes 41.

Ultraschallkopf 41, erster Laser 42 und zweiter Laser 43 sind beweglich und lösbar fixierbar in einer Aufhängung untergebracht (nicht gezeigt). Die Aufhängung ist mit einer Steuerung versehen, die über Elektromotoren den Ultraschallkopf 41 und die beiden Laser 42 und 43 bewegen kann. Der Ultraschallkopf 41 verfügt über Lagesensoren; diese übermitteln die gemessenen Lagedaten an die Steuerung, welche die Ausrichtung des Lasers 42 dann erfindungsgemäß an die Lage des Ultraschallkopfes 41 koppelt. Da hier weder Ultraschallkopf 41 noch einer der beiden Laser 42 und 43 per Hand bewegt werden müssen und die Lage des Ultraschallkopfes 41 gemessen wird bzw. die Steuerung ohnehin "weiß" in welcher Lage sich die Bestandteile des Systems 40 befinden, kann das System 40 auch von einem anderen Ort ferngesteuert werden.

Dazu kann das System 40 in einer virtuellen Realität nachgebildet und der Ultraschallkopf 41 über einen Datenhandschuh manipuliert werden.

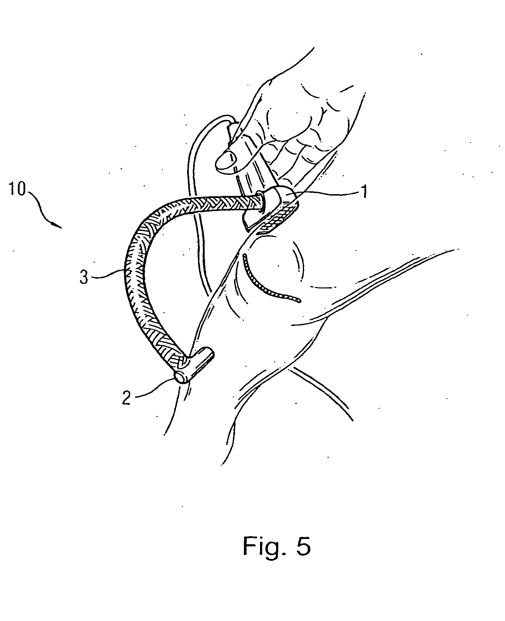

Figur 5 zeigt das System 10 aus Figur 1 angelegt an ein Knie.

## Patentansprüche

1. System (10, 40) zur geführten Sonographie mit
einem Ultraschallkopf (1, 41), der zur Ausführung einer Sonografie an die Oberfläche eines zu sonografierenden Objekts anlegbar und dort in seiner Lage veränderbar ist,
einem Projektor (2, 42) zur Projektion (5) einer von einer Bedienperson erkennbaren Markierung (31) auf das zu sonografierende Objekt,
einer Kopplungseinrichtung (3), die die Projektion (5) an die Lage des Ultraschallkopfes (1, 41) koppelt, und
einer auf dem zu sonografierenden Objekt von einer Bedienperson erkennbaren Referenz, deren Position und Ausbildung unabhängig von der Markierung ist,
wobei die Markierung in Bezug auf diese Referenz eine vorgegebene Position und dort eine vorgegebene Ausbildung auf dem zu sonografierenden Objekt aufweist, wenn der Ultraschallkopf (1, 41) an diesem korrekt anliegt.

2. System (10, 40) nach Anspruch 1, bei dem die Projektionsrichtung veränderlich ist und an die Lage des Ultraschallkopfes (1, 41) gekoppelt ist.

3. System (10, 40) nach Anspruch 1 oder 2, bei dem der Projektor (2, 42) ein Laser ist.

4. System (10, 40) nach einem der vorangehenden Ansprüche, bei dem die Markierung (31) ein Kreuz (31) ist.

5. System (10, 40) nach einem der vorangehenden Ansprüche, bei dem die relative Lage von dem Projektor (2, 42) zu dem Ultraschallkopf (1, 41) veränderlich und zur Einstellung lösbar festlegbar ist.

6. System (10, 40) nach einem der vorangehenden Ansprüche, bei dem die Kopplungseinrichtung (3) eine Messeinrichtung zur Messung der Lage des Ultraschallkopfes und eine Steuereinrichtung zur Steuerung des Projektors (2, 42), basierend auf der Messung der Lage des Ultraschallkopfes (1, 41) aufweist.

7. System (10, 40) nach einem der Ansprüche 1 bis 5, bei dem der Projektor (2, 42) und der Ultraschallkopf (1, 41) an einem gemeinsamen Träger (3) befestigt sind, so dass eine Lageänderung des Ultraschallkopfes (1, 41) über den Träger (3) eine Lageänderung des Projektors (2, 42) bewirkt.

8. System (10, 40) nach Anspruch 7, bei dem der Träger (3) ein zumindest abschnittweise U-förmiger Bügel (3) ist.

9. System (10, 40) nach Anspruch 7 oder 8, bei dem der Träger (3) austauschbar ist.

10. System (10, 40) nach einem der Ansprüche 7 bis 9, bei dem der Projektor (2, 42) entlang des Trägers (3) verschiebbar und lösbar fixierbar ist.

11. System (10, 40) nach einem der vorangehenden Ansprüche, mit einem zweiten Projektor (43) zur Projektion einer Referenzmarkierung (30) auf das zu sonographierende Objekt.

12. Träger (3) mit einem System (10, 40) zur geführten Sonographie nach Anspruch 9, auch in Kombination mit einem der weiteren Systemansprüche, zur Anpassung des Systems (10, 40) an bestimmte Anwendungsumstände.

## Claims

1. A system (10, 40) for guided sonography, comprising
an ultrasonic head (1, 41) which for carrying out a sonography can be applied to the surface of an object to be sonographized and is variable there in its position,
a projector (2, 42) for the projection (5) of a mark (31) recognizable by an operator onto the object to be sonographized,
a coupling means (3) which couples the projection (5) to the position of the ultrasonic head (1, 41), and
a reference recognizable by an operator on the object to be sonographized, whose position and formation is independent of the mark,
wherein the mark has a specified position with respect to this reference and at this position has a specified formation on the object to be sonographized, when the ultrasonic head (1, 41) is correctly applied to the same.

2. The system (10, 40) according to claim 1, in which the projection means is variable and is coupled to the position of the ultrasonic head (1, 41).

3. The system (10, 40) according to claim 1 or 2, in which the projector (2, 42) is a laser.

4. The system (10, 40) according to any of the preceding claims, in which the mark (31) is a cross (31).

5. The system (10, 40) according any of the preceding claims, in which the relative position from the projector (2, 42) to the ultrasonic head (1, 41) is variable and can releasably be fixed for adjustment.

6. The system (10, 40) according to any of the preceding claims, in which the coupling means (3) includes a measuring means for measuring the position of the ultrasonic head and a control means for controlling the projector (2, 42), based on the measurement of the position of the ultrasonic head (1, 41).

7. The system (10, 40) according to any of claims 1 to 5, in which the projector (2, 42) and the ultrasonic head (1, 41) are attached to a common carrier (3), so that a change in position of the ultrasonic head (1, 41) effects a change in position of the projector (2, 42) via the carrier (3).

8. The system (10, 40) according to claim 7, in which the carrier (3) is an at least partly U-shaped bow (3).

9. The system (10, 40) according to claim 7 or 8, in which the carrier (3) is replaceable.

10. The system (10, 40) according to any of claims 7 to 9, in which the projector (2, 42) is shiftable along the carrier (3) and can releasably be fixed.

11. The system (10, 40) according to any of the preceding claims, comprising a second projector (43) for the projection of a reference mark (30) onto the object to be sonographized.

12. A carrier (3) with a system (10, 40) for the guided sonography according to claim 9, also in combination with any of the further system claims, for adaptation of the system (10, 40) to certain applications.

## Revendications

1. Système (10, 40) pour sonographie guidée, comprenant une tête à ultrasons (1, 41) qui peut être appliquée sur la surface d'un objet à sonographier pour exécuter une sonographie et qui est peut être modifiée en position sur cette surface,
un projecteur (2, 42) pour la projection (5) sur l'objet à sonographier d'une marque (31) reconnaissable par un opérateur,
un système de couplage (3) qui couple la projection (5) à la position de la tête à ultrasons (1, 41), et
une référence sur l'objet à sonographier, reconnaissable par un opérateur, dont la position et la configuration sont indépendantes de la marque,
dans lequel la marque présente, par rapport à cette référence, une position prédéterminée et à cet endroit une conformation prédéterminée sur l'objet à sonographier quand la tête à ultrasons (1, 41) est correctement appliquée contre celui-ci.

2. Système (10, 40) selon la revendication 1, dans lequel la direction de projection est variable et est couplée à la position de la tête à ultrasons (1,41).

3. Système (10, 40) selon la revendication 1 ou 2, dans lequel le projecteur (2, 42) est un laser.

4. Système (10, 40) selon l'une des revendications précédentes, dans lequel la marque (31) est une croix (31).

5. Système (10, 40) selon l'une des revendications précédentes, dans lequel la position relative du projecteur (2, 42) par rapport à la tête à ultrasons (1, 41) est variable et peut être immobilisée de manière libérable pour le réglage.

6. Système (10, 40) selon l'une des revendications précédentes, dans lequel le système de couplage (3) comprend un moyen de mesure pour mesurer la position de la tête à ultrasons et un moyen de commande pour commander le projecteur (2, 42) en se basant sur la mesure de la position de la tête à ultrasons (1, 41).

7. Système (10, 40) selon l'une des revendications 1 à 5, dans lequel le projecteur (2, 42) et la tête à ultrasons (1, 41) sont fixés sur un support commun (3), de sorte qu'une modification de la position de la tête à ultrasons (1, 41) entraîne, via le support (3), une modification de position du projecteur (2, 42).

8. Système (10, 40) selon la revendication 7, dans lequel le support (3) est un arceau (3) présentant au moins par tronçons une forme en U.

9. Système (10, 40) selon la revendication 7 ou 8, dans lequel le support (3) est interchangeable.

10. Système (10, 40) selon l'une des revendications 7 à 9, dans lequel le projecteur (2, 42) est déplaçable en translation le long du support (3) et peut être fixé de manière détachable.

11. Système (10, 40) selon l'une des revendications précédentes, comprenant un second projecteur (43) pour la projection d'une marque de référence (30) sur l'objet à sonographier.

12. Support (3) avec un système (10, 40) pour la sonographie guidées selon la revendication 9, également en combinaison avec l'une des autres revendications concernant le système, pour l'adaptation du système (10, 40) à certaines particularités d'application.
